# EUROPÄISCHE PATENTSCHRIFT

(11) **EP 0 282 047 B1**
(45) Veröffentlichungstag und Bekanntmachung des Hinweises auf die Patenterteilung: **18.11.1993**
(21) Anmeldenummer: 88103759.2
(22) Anmeldetag: 10.03.1988
(51) Int. Cl.: A61N 1/05

(54) **Implantierbare Elektrodensonde mit ausfahrbarer Schraubelektrode**
Implantable probe with a protrudable cork screw electrode
Sonde implantable à électrode en spirale extractible

(30) Priorität: 13.03.1987 DE 3708133
(43) Veröffentlichungstag der Anmeldung: 14.09.1988
(73) Patentinhaber: Bisping, Hans-Jürgen, Dipl.-Ing., D-52072 Aachen (DE)
(72) Erfinder: Bisping, Hans-Jürgen, Dipl.-Ing., D-52072 Aachen (DE)
(74) Vertreter: Nau, Walter, Dipl.-Ing.

(56) Entgegenhaltungen:
- DE-A- 3 020 584
- US-A- 4 452 254

## Beschreibung

Die Patentanmeldung bezieht sich auf eine implantierbare Elektrondensonde mit einer flexiblen Zuleitung nach dem Oberbegriff von Patentanspruch 1.

Bei einer gattungsgemäßen Elektrodensonde (DE-OS 30 20 584) ist es bekannt, die den Speicher blockierende Sperrvorrichtung durch einen zusätzlichen vom proximalen Ende aus bedienbaren Sperrdraht bzw. -faden dadurch zu entriegeln, daß er aus der Elektrodensonde herausgezogen wird. Ein derartiger Sperrdraht (-faden) ist nur sehr schwer zu realisieren, da zum einem der dazu notwendige Raum fehlt, zum anderen aber größere Kräfte erforderlich sind, weil der Sperrdraht ein Teil der Sperrvorrichtung und daher eingeklemmt ist, so daß unerwünscht hohe Kräfte aufzubringen sind.

Weiterhin ist eine Elektrodensonde bekannt (DE-PS 25 33 766), die einen flexiblen, gewendelten Leiter aufweist, dessen distales Ende weitgewendelt ist, wobei der Leiter durch axialen Vorschub, dessen Kraft vom proximalen Ende her aufgebracht ist, herausschiebbar und einschraubbar ist. Da bei dieser gattungsfremden Elektrodensonde die zum axialen Vorschub erforderliche Kraft vom proximalen Ende aufgebracht werden muß, entsteht das Problem, daß aufgrund der Torsionskräfte entlang des elektrischen Leiters und der Isolierhülle eine Dislokation des Elektrodenkopfes während der Fixation erfolgen kann. Darüberhinaus ist eine derartige Manipulation bei der Fixation des Elektrodenkopfes unerwünscht.

Aufgabe der vorliegenden Erfindung ist es, die Nachteile der beschriebenen Elektrodensonden zu vermeiden und eine Elektrodensonde mit Sperrvorrichtung und Speicher derart zu verbessern, daß ein einfaches, zielsicheres Lösen der Sperrvorrichtung mit geringem Kraftaufwand möglich ist.

Die Aufgabe der Erfindung wird dadurch gelöst, daß der Verbindungskolben und der Elektrodenkopf alle zur Sperrung erforderlichen Elemente aufweisen und daß die Sperrvorrichtung durch axiale Bewegung des Verbindungskolbens innerhalb des Elektrodenkopfes lösbar ist. Durch diese Ausgestaltung der Sperrvorrichtung genügen kleine Axialkräfte, um ein Lösen der Sperrvorrichtung zu bewirken.

Die axiale Bewegung der Fixationsspirale bzw. des Verbindungskolbens kann dabei in beliebiger Weise mechanisch, hydraulisch oder pneumatisch, z.B. durch Kohlensäure, erfolgen.

Das Lösen der Sperrvorrichtung kann dabei mechanisch in einfacher Weise durch eine axiale Schubbewegung eines Mandrins erfolgen. Solche Mandrins werden üblicherweise in das Innere der Elektrodensonde eingeführt, um eine Stabilisierung der Elektrodensonde während der Implantierphase zu gewährleisten. Der dabei zumeist ohnehin vorhandene Mandrin kann daher zum Lösen der Sperrvorrichtung benutzt werden.

Besonders geeignet sind Sperrvorrichtungen nach den Ansprüchen 4 bis 8, die formschlüssig ineinandergreifende Verbindungselemente an der Fixationsspirale bzw. dem Verbindungskolben und dem Elektrodenkopf bzw. der Isolierhülle aufweisen, wobei insbesondere das der Fixationsspirale abgewandte Ende des Verbindungskolbens zur Aufnahme der Sperrvorrichtung geeignet ist.

Die Sperrvorrichtung kann aus einer Nut und einem Stift, aus einer sägezahnförmigen Ausnehmung und einem Stift sowie zueinanderpassenden sägezahnförmigen Ausnehmungen bzw. Vorsprüngen gebildet sein. Es sei darauf hingewiesen, daß auch alle anderen formschlüssigen Verbindungen, wie z.B. radial ineinandergreifende Keilprofile usw. möglich sind.

Bei den vorgeschlagenen Sperrvorrichtungen kann die für das Lösen der Sperrvorrichtung erforderliche Axialkraft durch entsprechende Neigungen der Anlageflächen der Sperrvorrichtungen zueinander beliebig eingestellt werden. Es empfiehlt sich natürlich die Neigung der Anlageflächen so zu wählen, daß die Sperrvorrichtung nur gewollt gelöst wird und sich nicht ungewollt von selbst löst. Es kann daher von Vorteil sein, wenn die Anlageflächen der Sperrvorrichtung derart geneigt sind, daß sie das Lösen der Sperrvorrichtung hemmen, wobei die Sperrelemente einen gewissen Verhakungseffekt in Verbindung mit der Torsionsspannung des Speichers erzeugen.

Damit nach dem Lösen der Sperrvorrichtung eine sichere Wirkverbindung zwischen dem Führungsteil bzw. dem Führungsstift und der Fixationsspirale entsteht, ist es erforderlich, die axiale Erstreckung der Fixationsspirale bzw. des Verbindungskolbens und die axiale Erstreckung der Sperrvorrichtung sowie die Lage des Führungsteils bzw. Führungsstifts so aufeinander abzustimmen, daß der Führungsstift in die Fixationsspirale eingreift, bevor die Sperrvorrichtung gelöst ist. Der Führungsstift hat dabei den Vorteil gegenüber üblichen anderen Führungsteilen, daß er einen zuverlässigen Eingriff in die Fixationsspirale bei geringen Reibkräften gewährleistet.

In vorteilhafter Weise kann der elektrische Leiter der Elektrodensonde selbst als Speicher ausgebildet sein, wobei eine drehfeste Verbindung des elektrischen Leiters an einer beliebigen Stelle der Isolierhülle erfolgen kann. Ist der elektrische Leiter am proximalen Ende der Elektrodensonde drehfest aber lösbar, beispielsweise mit einer Stiftschraube an der Isolierhülle befestigt, so kann die Torsionsspannung vor der Implantation der Elektrode aber auch während der Implantation vom proximalen Ende her aufgebracht werden. Ist die Drehsicherung des elektrischen Leiters an einer beliebigen Stelle der Isolierhülle vorgesehen und nicht von außen zugänglich, so kann die erforderliche Torsionsspannung durch eine Ansetzen eines Drehwerkzeuges in einer Eingriffsöffnung an der Fixationsspirale bzw. des Verbindungskolbens vom Elektrodenkopf her aufgebracht werden.

Um beim Implantieren ein ungewolltes Auslösen der Sperrvorrichtung zu verhindern, wird in vorteilhafter Weise der Mandrin dadurch gegen ein unbeabsichtigtes Vorschieben gesichert, daß am proximalen Ende des Mandrins ein Sicherungselement angebracht ist.

Zur weiteren Erläuterung der Erfindung wird auf die Zeichnungen verwiesen, in denen verschiedene Ausführungen der Erfindung vereinfacht dargestellt sind.
Es zeigen:
- Fig. 1: eine Ansicht eines distalen Endes der Elektrodensonde mit Teilschnitt durch den Elektrodenkopf, wobei die Fixationsspirale bzw. der Verbindungskolben in Sperrstellung gelagert sind;
- Fig. 2: eine Teilansicht des Verbindungskolbens;
- Fig. 3: eine Stirnansicht des Elektrodenkopfes;
- Fig. 4: eine Ansicht des distalen Endes der Elektrodensonde mit Schnitt durch den Elektrodenkopf gemäß Fig. 1, bei der jedoch die Fixationsspirale bzw. der Verbindungskolben aus der Sperre gelöst und herausgeschraubt ist;
- Fig. 5: eine weitere Teilansicht des Verbindungskolbens mit Schnitt durch einen am Elektrodenkopf angebrachten Stift;
- Fig. 6: eine Teilansicht des Verbindungskolbens mit anders gearteten Sperrelementen am Verbindungskolben und am Elektrodenkopf.

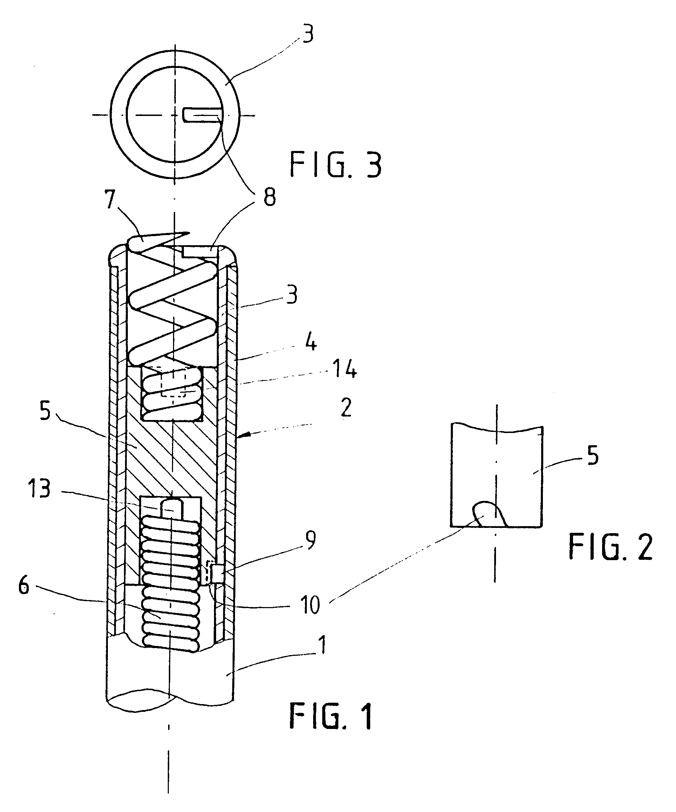

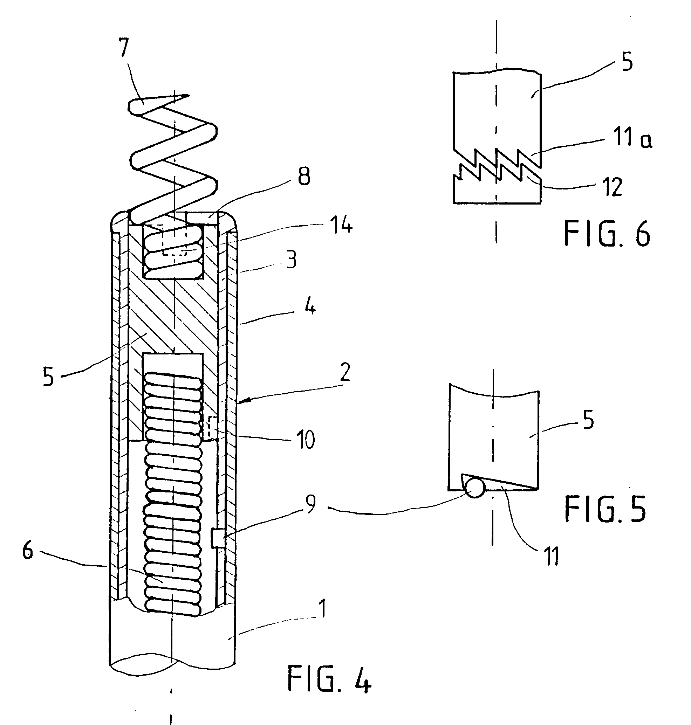

In den Figuren 1 bis 6 ist, soweit im einzelnen dargestellt, mit 1 das distale Ende einer Elektrodensonde bezeichnet, die einen mit 2 bezeichneten Elektrodenkopf aufweist. Der Elektrodenkopf 2 weist im Inneren eine elektrisch leitende Hülse 3 auf, die am distalen Ende des Elektrodenkopfes als Kontaktfläche ausgebildet ist. Die Hülse 3 ist von einer Isolierhülle 4 umgeben. Innerhalb der Hülse 3 ist ein Verbindungskolben 5 axial verschiebbar gelagert, der einerseits mit einem elektrischen Leiter 6 verbunden und am distalen Ende mit einer Fixationsspirale 7 versehen ist. Es sei darauf hingewiesen, daß der Verbindungskolben über Dichtringe in der Hülse geführt ist, wobei die Dichtringe auch aus leitendem Material hergestellt sein können, so daß ein guter elektrischer Kontakt zwischen dem elektrischen Leiter 6 und der Hülse 3 bzw. der Fixationsspirale 7 gewährleistet ist. Am distalen Ende der Hülse 3 ist ein nach innen ragender Führungsstift 8 befestigt, der nach Axialbewegung der Fixationsspirale bzw. des Verbindungskolbens aus seiner Sperrstellung in die Fixationsspirale eingreift und dieser eine Drehbewegung aufzwingt. Innerhalb der Hülse 3 ist weiterhin ein Stift 9 befestigt, der in Sperrstellung in eine Nut 10 des Verbindungskolbens 5 eingreift und diesen gegen Drehbewegung sperrt. Der Stift 9 kann aber auch, wie in Fig. 5 gezeigt, in eine sägezahnförmige Ausnehmung 11 am Verbindungskolben eingreifen und dadurch ebenfalls eine Drehsicherung bewirken.

Die Sperrvorrichtung kann aber auch, wie in Fig. 6 dargestellt, aus eine Vielzahl von sägezahnförmigen Ausnehmungen 11a am Verbindungskolben und 12 an der Hülse 3 bzw. einem Hilfselement der Hülse 3 bestehen.

Wie in Fig. 1 weiter dargestellt, ist innerhalb des elektrischen Leiters 6 ein Mandrin 13 gelagert, der mit seiner Stirnfläche am Verbindungskolben anliegt. Wird auf den Mandrin 13 vom proximalen Ende der Elektrodensonde her eine Axialkraft aufgebracht, so schiebt dieser den Verbindungskolben 5 aus der Sperrstellung, so daß dann die im elektrischen Leiter 6 gespeicherte Torsionskraft dem Verbindungskolben 5 und damit der Fixationsspirale 7 eine Drehbewegung aufzwingt, die dann in Verbindung mit dem Führungsstift 8 zu einem Herausdrehen der Fixationsspirale und des Verbindungskolbens führt.

Weiterhin weist der Verbindungskolben an seinem distalen Ende eine Eingriffsöffnung 14 für ein Drehwerkzeug auf, mittels der der elektrische Leiter gespannt werden kann.

Es sei noch darauf hingewiesen, daß im elektrischen Leiter 6 oder einem anderen Torsionsspeicher eine solche Grundspannung aufgebracht wird, daß die Fixationsspirale 7 bzw. der Verbindungskolben 5 in herausgeschraubter Stellung so fest am Führungsstift 8 anliegt, daß dort kein Wackelkontakt auftritt.

## Patentansprüche

1. Implantierbare Elektrodensonde mit einer flexiblen Zuleitung, die aus einer Isolierhülle (4) und einem in der Isolierhülle (4) gelagerten elektrischen Leiter (6) besteht, mit einem Elektrodenkopf (2, 3), der eine an einem Verbindungskolben (5) befestigte Fixationsspirale (7) aufweist, wobei die Fixationsspirale (7) über den Verbindungskolben (5) mit dem elektrischen Leiter (6) verbunden ist, mit einer lösbaren Sperrvorrichtung zwischen dem Verbindungskolben (5) und dem Elektrodenkopf (2, 3) und mit einem eine Drehenergie enthaltenden Speicher, der durch die Sperrvorrichtung in gespanntem Zustand blockierbar und mit dem elektrischen Leiter (6) und der Isolierhülle (4) derart verbindbar ist, daß sich die Fixationsspirale (7) mit Hilfe eines mit dem Elektrodenkopf (2, 3) verbundenen Führungsteils nach Lösen der Sperrvorrichtung herausdreht,
***dadurch gekennzeichnet,*** daß der Verbindungskolben (5) und der Elektrodenkopf (2, 3) alle zur Sperrung erforderlichen Elemente aufweisen und daß die Sperrvorrichtung durch axiale Bewegung des Verbindungskolbens (5) innerhalb des Elektrodenkopfes (2, 3) lösbar ist.

2. Implantierbare Elektrodensonde nach Anspruch 1,
***dadurch gekennzeichnet,*** daß die axiale Bewegung des Verbindungskolbens (5) durch mechanische, hydraulische oder pneumatische, z.B. Kohlensäure, Krafteinwirkung erfolgt.

3. Implantierbare Elektrodensonde nach Anspruch 1 oder 2,
***dadurch gekennzeichnet,*** daß die Sperrvorrichtung durch axialen Vorschub eines Mandrins (13) lösbar ist.

4. Implantierbare Elektrodensonde nach einem der vorhergehenden Ansprüche,
***dadurch gekennzeichnet,*** daß die Sperrvorrichtung formschlüssig ineinandergreifende Verbindungsteile an dem Verbindungskolben (5) und dem Elektrodenkopf (2, 3) aufweist.

5. Implantierbare Elektrodensonde nach einem der vorhergehenden Ansprüche,
***dadurch gekennzeichnet,*** daß die Sperrvorrichtung am der Fixationsspirale (7) abgewandten Ende des Verbindungskolbens (5) angeordnet ist.

6. Implantierbare Elektrodensonde nach einem der vorhergehenden Ansprüche,
***dadurch gekennzeichnet,*** daß die Sperrvorrichtung aus einer im Verbindungskolben (5) angeordneten Nut (10) und einem am Elektrodenkopf (2, 3) gelagerten Stift (9) besteht.

7. Implantierbare Elektrodensonde nach einem der vorhergehenden Ansprüche,
***dadurch gekennzeichnet,*** daß die Sperrvorrichtung aus einer sägezahnförmigen Ausnehmung (11) am Verbindungskolben (5) und einem Stift (9) am Elektrodenkopf (2, 3) besteht.

8. Implantierbare Elektrodensonde nach einem der vorhergehenden Ansprüche,
***dadurch gekennzeichnet,*** daß die Sperrvorrichtung aus sägezahnförmigen Ausnehmungen (11a bzw. 12) oder Vorsprüngen am Verbindungskolben (5) und am Elektrodenkopf (2, 3) besteht.

9. Implantierbare Elektrodensonde nach einem der vorhergehenden Ansprüche,
***dadurch gekennzeichnet,*** daß die Anlageflächen der Sperrvorrichtung derart geneigt sind, daß der Verbindungskolben (5) unter Axialkraft in der Verriegelungsstellung gehalten ist.

10. Implantierbare Elektrodensonde nach einem der vorhergehenden Ansprüche,
***dadurch gekennzeichnet,*** daß das Führungsteil als radial ausgerichteter Führungsstift (8) ausgebildet ist, der am distalen Endbereich des Elektrodenkopfes (2, 3) befestigt ist.

11. Implantierbare Elektrodensonde nach einem der vorhergehenden Ansprüche,
***dadurch gekennzeichnet,*** daß die axiale Erstreckung der Fixationsspirale (7), des Verbindungskolbens (5) und der Sperrvorrichtung sowie die Lage des Führungsstiftes (8) so aufeinander abgestimmt sind, daß der Führungsstift (8) in die Fixationsspirale (7) eingreift, bevor die Sperrvorrichtung gelöst ist.

12. Implantierbare Elektrodensonde nach einem der vorhergehenden Ansprüche, bei der der elektrische Leiter (6) als Speicher ausgebildet ist und am proximalen Ende der Elektrodensonde drehfest mit der Isolierhülle (4) verbunden ist,
***dadurch gekennzeichnet,*** daß die Speicherenergie durch Drehen am distalen Ende des Verbindungskolbens (5) aufbringbar ist.

13. Implantierbare Elektrodensonde nach einem der vorhergehenden Ansprüche,
***dadurch gekennzeichnet,*** daß der Verbindungskolben (5) eine Eingriffsöffnung (14) für ein Drehwerkzeug aufweist.

14. Implantierbare Elektrodensonde nach einem der vorhergehenden Ansprüche, bei dem der Mandrin am proximalen Ende mit einem Betätigungsknopf versehen ist,
***dadurch gekennzeichnet,*** daß bei ungelöster Sperrvorrichtung zwischen Betätigungsknopf des Mandrins (13) und der Elektrodensonde (1) eine Abstandssicherung angebracht ist.

## Claims

1. An implantable electrode probe having a flexible lead and comprising an insulating sheath (4), an electric conductor (6) mounted in the sheath (4), an electrode head (2, 3) having a fixing spiral (7) secured to a connecting piston (5), the spiral (7) being connected to the electric conductor (6) via the piston (5); a releasable locking device between the piston (5) and the electrode head (2, 3) and a storage unit containing rotary energy and lockable by the locking device in a state under tension and connectable to the electric conductor (6) and the sheath (4) so that,after the locking device is released, the spiral (7) is released by rotating, by means of a guide part connected to the electrode head (2, 3), characterised in that the connecting piston (5) and the electrode head (2, 3) comprise all the elements needed for locking, and the locking device is releasable by axial motion of the connecting piston (5) inside the electrode head (2, 3).

2. An implantable electrode probe according to claim 1, characterised in that the axial motion of the connecting piston (5) is brought about by mechanical, hydraulic or pneumatic force, e.g. carbon dioxide.

3. An implantable electrode probe according to claim 1 or 2, characterised in that the locking device is releasable by axial advance of a mandrel (13).

4. An implantable probe according to any of the preceding claims, characterised in that the locking device has positively-engaging connecting parts on the connecting piston (5) and the electrode head (2, 3).

5. An implantable probe according to any of the preceding claims, characterised in that the locking device is disposed on the end of the connecting piston (5) remote from the fixing spiral (7).

6. An implantable probe according to any of the preceding claims, characterised in that the locking device comprises a groove (10) formed in the connecting piston (5) and a pin (9) mounted on the electrode head (2, 3).

7. An implantable probe according to any of the preceding claims, characterised in that the locking device comprises a sawtooth recess (11) on the connecting piston (5) and a pin (9) on the electrode head (2, 3).

8. An implantable probe according to any of the preceding claims, characterised in that the the locking device comprises sawtooth recesses (11a, 12) or projections on the connecting piston (5) and on the electrode head (2, 3).

9. An implantable probe according to any of the preceding claims, characterised in that the abutment surfaces of the locking device are inclined so that the connecting piston (5) is held in the locked position by axial force.

10. An implantable probe according to any of the preceding claims, characterised in that the guide part is a radially-extending guide pin (8) secured to the distal end region of the electrode head (2, 3).

11. An implantable probe according to any of the preceding claims, characterised in that the axial length of the fixing spiral (7), the connecting piston (5) and the locking device and the position of the guide pin (8) are adjusted to one another so that the guide pin (8) engages in the spiral (7) before the locking device has been released.

12. An implantable probe according to any of the preceding claims, in which the electric conductor (6) is in the form of a storage unit and is non-rotatably connected to the insulating sheath (4) at the proximal end of the electrode probe, characterised in that the energy in the storage unit can be applied by rotation at the distal end of the connecting piston (5).

13. An implantable probe according to any of the preceding claims, characterised in that the connecting piston (5) has an opening (14) for engagement of a rotary tool.

14. An implantable probe according to any of the preceding claims, in which the mandrel is provided with an actuating button at its proximal end, characterised in that,when the locking device is not released, a spacer is provided between the probe (1) and the actuating head of the mandrel (13).

## Revendications

1. Sonde à électrode implantable comportant une conduite d'alimentation souple formée d'une douille isolante (4) dans laquelle se trouve un conducteur électrique (6), avec une tête d'électrode (2, 3) ayant une spirale de fixation (7) fixée sur un piston de liaison (5), la spirale de fixation (7) étant reliée au conducteur électrique (6) par l'intermédiaire du piston de liaison (5), un dispositif de blocage desserrable étant prévu entre le piston de liaison (5) et la tête d'électrode (2, 3) avec un accumulateur dans lequel est emmagasinée de l'énergie de rotation, qui peut être bloqué dans cet état par le dispositif de blocage et peut être relié au conducteur électrique (6) et à la douille isolante (4) de façon que la spirale de fixation (7) se dévisse vers l'extérieur après desserrage du dispositif de blocage à l'aide d'une pièce de guidage reliée à la tête d'électrode (2, 3), caractérisée en ce que le piston de liaison (5) et la tête d'électrode (2, 3) comportent tous les éléments nécessaires au blocage, et en ce que le dispositif de blocage peut se desserrer par mouvement axial du piston de liaison (5) dans la tête d'électrode (2, 3).

2. Sonde à électrode implantable selon la revendication 1, caractérisée en ce que le mouvement axial du piston de liaison (5) est assuré par action d'une force mécanique, hydraulique ou pneumatique, par exemple par du gaz carbonique.

3. Sonde à électrode implantable selon la revendication 1 ou 2, caractérisée en ce que le dispositif de blocage peut être desserré par l'avancée axiale d'un mandrin (13).

4. Sonde à électrode implantable selon l'une des revendications précédentes, caractérisée en ce que le dispositif de blocage comporte des pièces de liaison venant prendre l'une dans l'autre par une liaison de forme sur le piston de liaison (5) et la tête d'électrode (2, 3).

5. Sonde à électrode implantable selon l'une des revendications précédentes, caractérisée en ce que le dispositif de blocage est prévu à l'extrémité du piston (5) éloignée de la spirale de fixation (7).

6. Sonde à électrode implantable selon l'une des revendications précédentes, caractérisée en ce que le dispositif de blocage se compose d'une rainure (10) dans le piston de liaison (5) et d'une tige (9) portée par la tête d'électrode (2, 3).

7. Sonde à électrode implantable selon l'une des revendications précédentes, caractérisée en ce que le dispositif de blocage se compose d'une cavité (11) en forme de dents de scie sur le piston de liaison (5) et d'une tige (9) sur la tête d'électrode (2, 3).

8. Sonde à électrode implantable selon l'une des revendications précédentes, caractérisée en ce que le dispositif de blocage se compose de cavités (11a, 12) en forme de dents de scie ou de saillies sur le piston de liaison (5) et sur la tête d'électrode (2, 3).

9. Sonde à électrode implantable selon l'une des revendications précédentes, caractérisée en ce que les surfaces d'appui du dispositif de blocage sont inclinées pour que le piston de liaison (5) soit maintenu en position de verrouillage par la force axiale.

10. Sonde à électrode implantable selon l'une des revendications précédentes, caractérisée en ce que la pièce de guidage est en forme de tige de guidage (8) alignée radialement et fixée à l'extrémité distale de la tête d'électrode (2, 3).

11. Sonde à électrode implantable selon l'une des revendications précédentes, caractérisée en ce que l'extension axiale de la spirale de fixation (7) du piston de liaison (5) et du dispositif de blocage ainsi que la position de la tige de guidage (8) sont accordées réciproquement pour que la tige de guidage (9) pénètre dans la spirale de fixation (7) avant le desserrage du dispositif de blocage.

12. Sonde à électrode implantable selon l'une des revendications précédentes, caractérisée en ce que le conducteur électrique (6) est en forme d'accumulateur et relié à la douille isolante (4) à l'extrémité proximale de la sonde à électrode, et en ce que l'énergie accumulée est appliquée par rotation de l'extrémité distale du piston de liaison (5).

13. Sonde à électrode implantable selon l'une des revendications précédentes, caractérisée en ce que le piston de liaison (5) comporte un orifice de prise (14) pour recevoir un outil de rotation.

14. Sonde à électrode implantable selon l'une des revendications précédentes, le mandrin étant muni d'un bouton de manoeuvre à son extrémité proximale, sonde caractérisée en ce que lorsque le dispositif de blocage n'est pas desserré, on prévoit une sécurité d'écartement entre le bouton de manoeuvre du mandrin (13) et la sonde à électrode (1).
